(19) Europäisches Patentamt
European Patent Office
Office européen des brevets

(11) Publication number: **0 056 475**
A2

# (12) EUROPEAN PATENT APPLICATION

(21) Application number: 81110638.4

(22) Date of filing: 21.12.81

(51) Int. Cl.³: C 07 D 285/00
C 07 D 521/00, A 61 K 31/41

(30) Priority: 13.01.81 US 224715

(43) Date of publication of application:
28.07.82 Bulletin 82/30

(84) Designated Contracting States:
AT BE CH DE FR GB IT LI NL SE

(71) Applicant: Merck Patent Gesellschaft mit beschränkter Haftung
Frankfurter Strasse 250
D-6100 Darmstadt(DE)

(72) Inventor: Jacobi, Ernst, Dr.
Rosenhöhweg 14
D-6100 Darmstadt(DE)

(72) Inventor: Gericke, Rolf, Dr.
Mozartstrasse 19
D-6104 Seeheim-Jugenheim(DE)

(72) Inventor: Coutts, Stephen M., Dr.
61 Walworth Avenue
Scarsdale New York 10583(US)

## (54) Pharmaceutical compositions.

(57) Pharmaceutical compositions comprising thiazathiolium derivatives of the formula

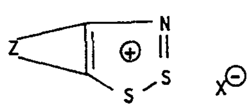

wherein 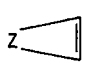

stands for an aromatic or heteroaromatic system which may be substituted or unsubstituted and $X^{\ominus}$ is $OH^{\ominus}$ or an anion of a strong acid, are useful as antiallergic agents.

EP 0 056 475 A2



COMPLETE DOCUMENT

## SPECIFICATION

The present invention relates to novel pharmaceutical compositions based on thiazathiolium derivatives as well as to novel compounds and methods of effecting therapeutic activities, e.g. antiallergic.

## SUMMARY OF THE INVENTION

It is an object of one aspect of this invention to provide novel compounds. Another aspect is to provide novel pharmaceutical compositions which can be used as medicaments. Still other objects include methods of effecting pharmaceutical activities.

Upon further study of the specification and appended claims, further objects and advantages of this invention will become apparent to those skilled in the art.

The pharmaceutical compositions of this invention comprise compounds of Formula I

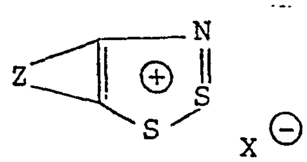

(I)

wherein

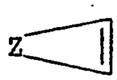 stands for an aromatic or heteroaromatic system which may be substituted or unsubstituted and

$X^{\ominus}$ is $OH^{\ominus}$ or an anion of a strong acid.

BACKGROUND OF THE INVENTION

Some compounds of Formula I are known, particularly some of those wherein X is Cl ("Herz compounds").

Some of these known compounds have been used as post-emergent herbicides and dyestuff intermediates as described in U.S. Patents No. 3,490,893 and 3,808,202 and references cited therein (cf. also The Merck Index, ninth edition, 1976, Merck & Co., Inc., Rahway, N.J., Organic Name Reactions, page ONR-42, "Herz-Reaction", and references cited therein), all said patents and references being incorporated herein.

In German Patent No. 487,849, filed May 15, 1923 by Leopold Casella & Co., there is mentioned in Example 3(C) a compound of the formula:

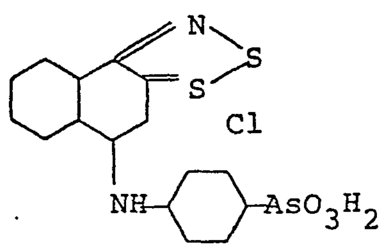

obtained by condensation of arsanilic acid with the product obtained from 1-naphthylamine and $S_2Cl_2$.

The above compound is indicated for a therapeutic use, broadly speaking, but without any specifics whatsoever as to the type of therapy or how it is to be administered. In any case, this compound is excluded from the pharmaceutical compositions of this invention which are not based on any field of intended therapy. Conversely, where a specific utility is associated with a pharmaceutical composition or method of administration, this compound is intended to be included among the active ingredients.

## DETAILED DISCUSSION

In Formula I, 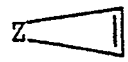 stands preferably for a benzene ring (benzo group) which may be substitued. The group 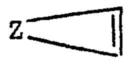 may also stand for a naphthalene ring (1,2-, 2,3- or 3,4-naphtho group), a thiophene ring (1,2- or 2,1-thieno group), furthermore for one of the following rings: phenanthrene, anthracene, pyridine, pyrrole, furan, quinoline, isoquinoline, indole, benzothiophene, benzofuran, carbazole, dibenzothiophene, dibenzofuran, pyrazine, pyrimidine, pyrazine, thiazine, phenothiazine.

$X^{\ominus}$ is preferably $OH^{\ominus}$ or halogenide, particularly chloride, but also fluoride, bromide, iodide, hydrogensulfate, perchlorate, nitrate, tetrafluoroborate.

Those compounds wherein $X^{\ominus}$ is $OH^{\ominus}$ can occur in various tautomeric forms such as

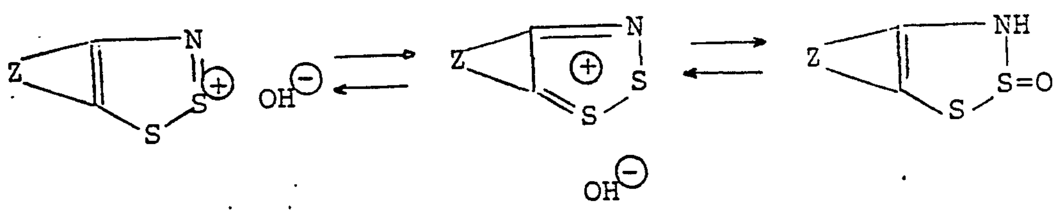

A B C

It appears that the 1,2,3-dithiazole-2-oxide form C is favored over its tautomers. All these tautomers are included in formula I which of course, encompasses tautomers of compounds wherein $X^{\ominus}$ stands for an anion of a strong acid.

Preferred compounds of Formula I are those wherein 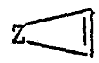 forms a benzene ring which may be substituted, particularly those of Formula Ia

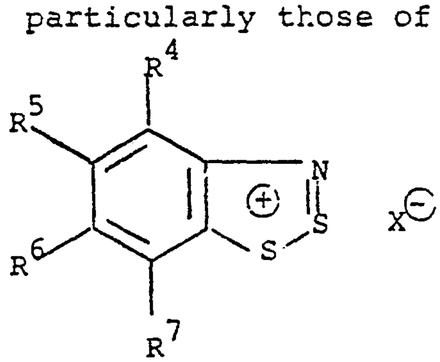

(Ia)

wherein

$R^4$ to $R^7$ are independently hydrogen atoms or substituents.

The compounds of Formula Ia are 1,3,2-benzothiazathiolium derivatives (cf. The Ring Index, 1960, American Chemical Society, No. 1052). They can also be written, however, in the form of 2,3,1-benzothiazathiolium derivatives (Formula D; The Ring Index, l.c., No. 1051)

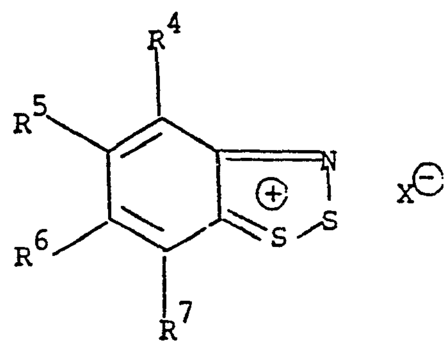

D

For some of the compounds of Formula Ia, particularly for the salts of 6-$R^1$NH- or 6-$R^2R^3$N-derivatives (wherein $R^1$, $R^2$ and $R^3$ are optionally substituted hydrocarbyl groups), the quinonoid structures E and F may also be considered:

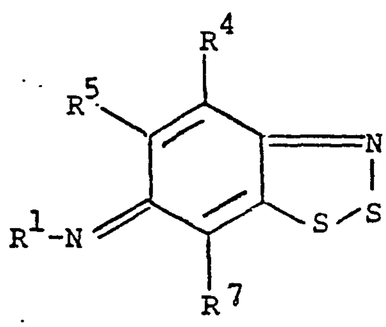

. HX

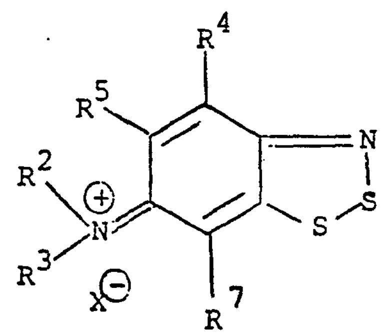

E F

All these structural tautomers (A to F) are included in Formulae I and Ia, respectively.

Furthermore, some of the bases corresponding to the salts of Formula E may occur in the "des-HX"-form of 6-$R^1$N-6H-1,2,3-benzodithiazoles (cf. The Ring-Index, Supplement I, 1963, American Chemical Society, No. 7932) of Formula G:

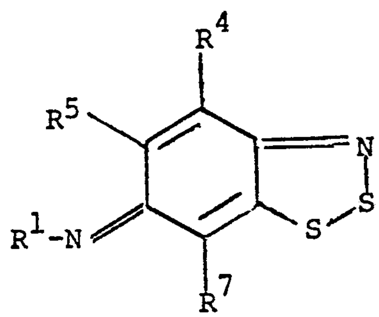

G

wherein $R^1$, $R^4$, $R^5$ and $R^7$ are as above.

The pharmaceutical compositions of this invention also comprise compounds of Formula G and their physiologically acceptable acid addition salts of Formula E.

Preferred substituents $R^4$ to $R^7$ are - independently - any of the following:

halo, preferably chloro, but also fluoro, bromo or iodo; nitro; amino; hydroxy;

alkyl of preferably 1 to 4 carbon atoms such as methyl, ethyl, furthermore n-propyl, isopropyl, n-butyl, isobutyl, sec.-butyl or tert.-butyl;

acyl of preferably 1 to 8 carbon atoms such as formyl, acetyl, propionyl or benzoyl;

alkoxy of preferably 1 to 4 carbon atoms such as methoxy, ethoxy, furthermore n-propyloxy, isopropyloxy, n-butyloxy, isobutyloxy, sec.-butyloxy, tert.-butyloxy;

mono- or polyhaloalkyl such as trifluoromethyl;

alkylthio of preferably 1 to 4 carbon atoms such as methylthio, ethylthio, furthermore n-propylthio, isopropylthio, n-butylthio, isobutylthio, sec.-butylthio, tert.-butylthio;

trifluoromethylthio;

alkylamino of preferably 1 to 4 carbon atoms such as methylamino, ethylamino, furthermore n-propylamino, isopropylamino, n-butylamino, isobutylamino, sec.-butylamino, tert.-butylamino;

dialkylamino of preferably 1 to 4 carbon atoms in each alkyl group such as dimethylamino, diethylamino, furthermore N-ethyl-N-methylamino, di-n-propylamino, or di-n-butylamino;

aryl of preferably 6 to 14 carbon atoms such as phenyl, 1- or 2-naphthyl;

substituted aryl of preferably 6 to 14 carbon atoms such as o-, m- or p-fluorophenyl, o-, m- or p-chlorophenyl, o-, m- or p-bromophenyl, o-, m- or p-iodophenyl;

aryloxy of preferably 6 to 14 carbon atoms such as phenoxy, 1- or 2-naphthoxy;

substituted aryloxy of preferably 6 to 14 carbon atoms such as o-, m- or p-fluorophenoxy, o-, m- or p-chlorophenoxy, o-, m- or p-bromophenoxy, o-, m- or p-iodophenoxy;

aryl-alkoxy of preferably 7 to 11 carbon atoms such as benzyloxy;

substituted aryl-alkoxy of preferably 7 to 11 carbon atoms such as o-, m- or p-chlorobenzyloxy;

arylthio of preferably 6 to 14 carbon atoms such as phenylthio, 1- or 2-naphthylthio;

substituted arylthio of preferably 6 to 14 carbon atoms such as o-, m- or p-fluorophenylthio, o-, m- or p-chlorophenylthio, o-, m- or p-bromophenylthio, o-, m- or p-iodophenylthio;

aryl-alkylthio of preferably 7 to 11 carbon atoms such as benzylthio;

substituted aryl-alkylthio of preferably 7 to 11 carbon atoms such as o-, m- or p-chlorobenzylthio;

cycloalkylamino of preferably 3 to 8 carbon atoms, such as cyclopropylamino, cyclobutylamino, cyclopentylamino, cyclohexylamino, cycloheptylamino or cyclooctylamino;

saturated cyclic amino of preferably 4 to 8 carbon atoms such as pyrrolidino, piperidino, morpholino, 4-alkyl-piperazino (wherein the alkyl group is preferably of 1 to 4 carbon atoms), f.e. 4-methyl-piperazino;

arylamino of preferably 6 to 14 carbon atoms, such as anilino, o-, m- or p-toluidino, 2,4,6-trimethylanilino, 1- or 2-naphthylamino, o-, m- or p-phenylanilino;

diarylamino of preferably 12 to 18 carbon atoms, such as diphenylamino;

substituted arylamino of preferably 6 to 14 carbon atoms, f.e. mono- or polyhalo-arylamino such as o-, m- or p-fluoroanilino, o-, m- or p-bromoanilino o-, m- or p-iodoanilino, 2,3-, 2,4-, 2,5-, 2,6-, 3,4- or 3,5-difluoroanilino, 2,3-, 2,4-, 2,5-, 2,6-, 3,4- or 3,5-dichloroanilino, 2,3-, 2,4-, 2,5-, 2,6-, 3,4- or 3,5-dibromoanilino, 2-chloro-4-fluoro-anilino, 4-chloro-2-fluoro-anilino, 4-bromo-2-chloro-anilino, 2-bromo-4-chloro-anilino, 2,3,4-, 2,3,5-, 2,3,6-, 2,4,5-, 2,4,6- or 3,4,5-trifluoro-anilino, 2,3,4-, 2,3,5-, 2,3,6-, 2,4,5-, 2,4,6- or 3,4,5-trichloroanilino, 2,3,4,5- or 2,3,4,6-tetrafluoroanilino, 2,3,4,5- or 2,3,4,6-tetrachloroanilino, pentafluoroanilino, pentachloroanilino; mono- or polynitroarylamino such as o-, m- or p-nitroanilino, 2,4-dinitroanilino; mono- or polyaminoarylamino such as o-, m- or p-aminoanilino; mono- or polyhydroxyarylamino such as o-, m- or p-hydroxyanilino, 2,3-, 2,4-, 2,5-, 2,6-, 3,4- or 3,5-dihydroxyanilino; sulfo-arylamino such as o-, m- or p-sulfo-anilino; mono- or polyalkoxyarylamino (wherein the alkoxy groups contain preferably 1 to 4 carbon atoms) such as o-, m- or p-methoxyanilino, o-, m- or p-ethoxyanilino,

2,3-, 2,4-, 2,5-, 2,6-, 3,4- or 3,5-dimethoxyanilino; carboxy-arylamino such as o-, m- or p-carboxyanilino;

N-alkyl-N-arylamino of preferably 7 to 11 carbon atoms such as N-methyl-anilino or N-ethyl-anilino;

substituted N-alkyl-N-arylamino of preferably 7 to 11 carbon atoms such as N-methyl-o-, -m- or -p-chloro-anilino, N-ethyl-o-, -m- or -p-chloroanilino;

arylalkylamino of preferably 7 to 11 carbon atoms such as benzylamino, 1- or 2-phenylethylamino, 1- or 2-naphthylmethylamino;

substituted arylalkylamino of preferably 7 to 11 carbon atoms, such as o-, m- or p-chlorobenzylamino;

heteroaryl-thio of preferably 3 to 12 carbon atoms such as 5-methyl-1,3,4-thiadiazol-2-ylthio, pyridyl-2-, -3- or -4-thio;

heteroaryl-amino of preferably up to 12 carbon atoms, such as 2-pyrimidylamino, 2- or 3-thienylamino, 2-, 3- or 4-pyridylamino, 2-, 3-, 4-, 5-, 6- or 7-benzo-thienylamino, 3,4-methylenedioxy-anilino;

acylamino of preferably 1 to 7 carbon atoms, such as formamido, acetamido or benzamido;

substituted acylamino of preferably 1 to 7 carbon atoms such as o-, m- or p-chlorobenzamido;

carboxyl; cyano;

alkoxycarbonyl of preferably 2 to 5 carbon atoms, such as methoxycarbonyl, ethoxycarbonyl or n-butoxycarbonyl.

Particularly preferred are those compounds of Formulae I and Ia which contain 0 to 4 of these preferred substituents.

Of the compounds of Formula Ia, preferred are those of Formulae Ib through Iz which correspond to Formula Ia with the radicals $R^4$ to $R^7$ having the following values, respectively:

Ib $R^4$ to $R^7$ are independently each H, F, Cl, Br, I, $NO_2$, $NH_2$, OH, alkyl of 1 to 4 carbon atoms, acyl of 1 to 8 C atoms, alkoxy of 1 to 4 carbon atoms, trifluoromethyl, alkylthio of 1 to 4 carbon atoms, trifluoromethylthio, alkylamino of 1 to 4 C atoms, dialkylamino of 1 to 4 C atoms, unsubstituted or substituted aryl of 6 to 14 C atoms, unsubstituted or substituted aryloxy of 6 to 14 C atoms,

unsubstituted or substituted aryl-alkoxy of 6 to 14 C atoms,

unsubstituted or substituted arylthio of 6 to 14 C atoms,

unsubstituted or substituted arylalkylthio of 6 to 14 C atoms,

cycloalkylamino of 3 to 8 C atoms, saturated cyclic amino of 4 to 8 C atoms, unsubstituted or substituted arylamino of 6 to 14 C atoms, unsubstituted or substituted

arylalkylamino of 7 to 11 C atoms, heteroarylamino of up to 12 C atoms, acylamino of 1 to 7 C atoms, COOH, CN, alkoxycarbonyl of 2 to 5 C atoms.

Ic $R^4$, $R^5$ and $R^7$ are H, F, Cl, $NO_2$, methyl, ethyl, methoxy, ethoxy, $CF_3$ or methylthio and

$R^6$ is H, F, Cl, $NO_2$, methyl, ethyl, methoxy, ethoxy, $CF_3$, methylthio, $NH_2$, alkylamino of 1 to 4 C atoms, dialkylamino of 1 to 4 C atoms, cycloalkylamino of 3 to 8 C atoms, saturated cyclic amino of 4 to 8 C atoms, unsubstituted or substituted arylamino of 6 to 14 C atoms, arylalkylamino of 7 to 11 C atoms, heteroarylamino of up to 12 C atoms, or acylamino of 1 to 7 C atoms.

Id $R^4$, $R^5$ and $R^7$ are H, F, Cl, $NO_2$, methyl or methoxy and

$R^6$ is $NH_2$, alkylamino of 1 to 4 C atoms, dialkylamino of 1 to 4 C atoms, cycloalkylamino of 3 to 8 C atoms,

saturated cyclic amino of 4 to 8 C atoms, unsubstituted or substituted arylamino of 6 to 14 C atoms,
arylalkylamino of 7 to 11 C atoms, heteroarylamino of up to 12 C atoms, or acylamino of 1 to 7 C atoms.

Ie $R^4$, $R^5$ and $R^7$ are H, F, Cl, $NO_2$, methyl or methoxy and
$R^6$ is unsubstituted or substituted arylamino of 6 to 14 C atoms;

If $R^4$, $R^5$ and $R^7$ are H, F, Cl, $NO_2$, methyl or methoxy and
$R^6$ is anilino, optionally substituted by 1 to 3 alkyl or alkoxy groups of 1 to 4 C atoms each, 1 or 2 hydroxy groups, $NH_2$, 1 to 3 halogen atoms, $SO_3H$, COOH or phenyl;

Ig $R^4$, $R^5$ and $R^7$ are H, F, Cl, $NO_2$, methyl or methoxy and
$R^6$ is anilino, optionally substituted by 1 or 2 halogen atoms or by OH;

Ih $R^4$, $R^5$ and $R^7$ are H, F, Cl, $NO_2$, methyl or methoxy and

$R^6$ is anilino substituted by 1 to 5 chloro atoms;

Ii $R^4$, $R^5$ and $R^7$ are H, F, Cl, $NO_2$, methyl or methoxy and

$R^6$ is anilino substituted by 1 to 5 fluoro atoms;

Ij $R^4$, $R^5$ and $R^7$ are H, F, Cl, $NO_2$, methyl or methoxy and

$R^6$ is anilino optionally substituted by 1 or 2 halogen atoms or by OH;

Ik $R^4$, $R^5$ and $R^7$ are H, F, Cl, $NO_2$, methyl or methoxy and

$R^6$ is hydroxyanilino;

Il $R^4$ is H or methyl,

$R^5$ is H,

$R^6$ is $NH_2$, alkylamino of 1 to 4 C atoms, dialkylamino of 1 to 4 C atoms, cycloalkylamino of 3 to 8 C atoms, saturated cyclic amino of 4 to 8 C atoms, unsubstituted or substituted arylamino of 6 to 14 C atoms, arylalkylamino of 7 to 11 C atoms, heteroarylamino of up to 12 C atoms, or acylamino of 1 to 7 C atoms, and

$R^7$ is H;

Im $R^4$ is H or methyl,
$R^5$ is H,
$R^6$ is unsubstituted or substituted arylamino of 6 to 14 C atoms, and
$R^7$ is H;

In $R^4$ is H or methyl
$R^5$ is H,
$R^6$ is anilino, optionally substituted by 1 to 3 alkyl or alkoxy groups of 1 to 4 C atoms each, 1 or 2 hydroxy groups, $NH_2$, 1 to 3 halogen atoms, $SO_3H$, COOH or phenyl, and
$R^7$ is H,

Io $R^4$ is H or methyl,
$R^5$ is H,
$R^6$ is anilino, optionally substituted by 1 or 2 halogen atoms or by OH, and
$R^7$ is H;

Ip $R^4$ is H or methyl,
$R^5$ is H,
$R^6$ is anilino substituted by 1 to 5 chloro atoms, and
$R^7$ is H;

Iq $R^4$ is H or methyl,

$R^5$ is H,

$R^6$ is anilino substituted by 1 to 5 fluoro atoms, and

$R^7$ is H;

Ir $R^4$ is H or methyl,

$R^5$ is H,

$R^6$ is anilino substituted by 1 to 3 bromo atoms, and

$R^7$ is H;

Is $R^4$ is H or methyl,

$R^5$ is H,

$R^6$ is hydroxyanilino, and

$R^7$ is H;

It $R^4$ is H or methyl,

$R^5$ is H,

$R^6$ is chloro-anilino and

$R^7$ is H;

Iu $R^4$ is methyl,

$R^5$ is H,

$R^6$ is anilino, optionally substituted by 1 to 3 alkyl or alkoxy groups of 1 to 4 C atoms each, 1 or 2 hydroxy groups, $NH_2$, 1 to 3 halogen atoms, $SO_3H$, COOH or phenyl, and

$R^7$ is H;

| | | |
|---|---|---|
| Iv | $R^4$ is | methyl, |
| | $R^5$ is | H, |
| | $R^6$ is | anilino optionally substituted by 1 or 2 halogen atoms or by OH, and |
| | $R^7$ is | H; |
| Iw | $R^4$ is | methyl, |
| | $R^5$ is | H, |
| | $R^6$ is | anilino optionally substituted by 1 or 2 chloro atoms, and |
| | $R^7$ is | H; |
| Ix | $R^4$ is | methyl, |
| | $R^5$ is | H, |
| | $R^6$ is | chloro-anilino, and |
| | $R^7$ is | H. |
| Iy | $R^4$,$R^5$ and $R^7$ are | H, F, Cl, $NO_2$, methyl or methoxy and |
| | $R^6$ is | Cl; |
| Iz | $R^4$ is | H or methyl, |
| | $R^5$ is | H, |
| | $R^6$ is | Cl and |
| | $R^7$ is | H. |

In detail, the following 1,3,2-benzothiazathiolium derivatives are particularly suitable for the purpose of this invention:

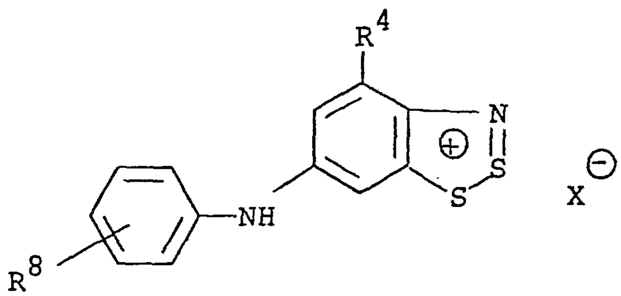

wherein

$R^4$ is H, F, Cl, Br, I, $NO_2$, OH, alkyl, alkoxy or alkylthio of 1 to 4 carbon atoms each or $CF_3$ and

$R^8$ is F, Cl, Br, I, OH, alkyl or alkoxy of 1 to 4 carbon atoms each or $CF_3$.

The p-position of $R^8$ is preferred.

Of the above group, compounds wherein

(a) $R^8$ is in the p-position and is F, Cl, Br or OH;

(b) $R^8$ is p-chloro;

(c) $R^8$ is $CF_3$;

(d) $R^4$ is F, Br, I, $NO_2$, OH, alkyl or alkoxy of 2-4 carbon atoms, alkylthio of 1-4 carbon atoms or $CF_3$, particularly those of (a), (b) or (c);

(e) $R^4$ is F, particularly those of (a), (b) or (c);

(f) $R^4$ is Br, particularly those of (a), (b) or (c);

(g) $R^4$ is I, particularly those of (a), (b) or (c);

(h) $R^4$ is $NO_2$, particularly those of (a), (b) or (c);

(i) $R^4$ is OH, particularly those of (a), (b) or (c);

(j) $R^4$ is alkyl or alkoxy of 2 to 4 carbon atoms each, particularly those of (a), (b) or (c);

(k) $R^4$ is alkylthio of 1 to 4 carbon atoms, particularly those of (a), (b) or (c);

(l) $R^4$ is methyl and $R^8$ is in the p-position and is F, Cl, Br or OH;

(m) $R^4$ is methyl and $R^8$ is $CF_3$;

are novel.

Also, the bases of Formula G corresponding to (a) to (m) are novel.

Among the bases of Formula G those are preferred which correspond to the preferred compounds of Formulae Ib to Ix.

In any case, this invention also contemplates the production and use of the following specific 1,3,2-thiazathiolium chlorides and also the corresponding hydroxides or salts, e.g., bromides, perchlorates and tetrafluoroborates, and their tautomers as well as the corresponding bases of Formula G:

unsubstituted 1,3,2-benzothiazathiolium chloride, 4-fluoro-, 5-fluoro-, 6-fluoro-, 7-fluoro-, 4-chloro-, 5-chloro-, 6-chloro-, 7-chloro-, 4-bromo-, 5-bromo-, 6-bromo-, 7-bromo-, 4-iodo-, 5-iodo-, 6-iodo-, 7-iodo-, 4-nitro-, 5-nitro-, 6-nitro-, 7-nitro-, 4-amino-, 5-amino-, 6-amino-, 7-amino-, 4-hydroxy-, 5-hydroxy-, 6-hydroxy-, 7-hydroxy-, 4-methyl-, 5-methyl-, 6-methyl-, 7-methyl-, 4-methoxy-, 5-methoxy-, 6-methoxy-, 7-methoxy-, 4-trifluoromethyl-, 5-trifluoromethyl-, 6-trifluoromethyl-, 7-trifluoromethyl-, 4-methylthio-, 5-methylthio-, 6-methylthio-, 7-methylthio-, 6-methylamino-, 4-formamido-, 5-formamido-, 6-formamido-, 7-formamido-, 4-ethyl-, 5-ethyl-, 6-ethyl-, 7-ethyl-, 4-ethoxy-, 5-ethoxy-, 6-ethoxy-, 7-ethoxy-, 4-ethylamino-, 5-ethylamino-, 6-ethylamino-, 7-ethylamino-, 4-methoxycarbonyl-, 5-methoxycarbonyl-,6-methoxycarbonyl-, 7-methoxycarbonyl-, 4-ethoxycarbonyl-, 5-ethoxycarbonyl-, 6-ethoxycarbonyl-, 7-ethoxycarbonyl-, 4-dimethylamino-, 5-dimethylamino-, 6-dimethylamino-, 7-dimethylamino-, 4-acetamido-, 5-acetamido-, 6-acetamido-, 7-acetamido-, 6-diethylamino-, 6-(5-methyl-1,3,4-thiadiazol-2-yl-thio)-, 6-pyrrolidino-, 6-piperidino-, 6-(4-methylpiperazino)-, 6-(N'-3-methylaza-pentamethylen)-hydrazino-, 6-(2-pyrimidylamino)-, 6-(4-pyridylthio)-, 6-(2-pyridylamino)-, 6-(3-pyridylamino)-, 6-(4-pyridylamino)-, 4-phenyl-, 5-phenyl-, 6-phenyl-, 7-phenyl-, 6-o-chlorophenyl-, 6-m-chlorophenyl-, 4-p-chlorophenyl-, 5-p-chlorophenyl-, 6-p-chlorophenyl-, 7-p-chlorophenyl-, 4-phenoxy-, 5-phenoxy-, 6-phenoxy-, 7-phenoxy-, 6-o-chlorophenoxy-, 6-m-chlorophenoxy-, 4-p-chlorophenoxy-, 5-p-chlorophenoxy-, 6-p-chlorophenoxy-, 7-p-chlorophenoxy-, 6-p-hydroxyphenoxy-, 6-(3,5-dihydroxyphenoxy)-, 6-phenylthio-, 6-p-chlorophenylthio-, 4-anilino-, 5-anilino-, 6-anilino-, 7-anilino-, 6-o-bromoanilino-, 6-m-bromoanilino-, 6-p-bromoanilino-, 6-o-chloroanilino-, 6-m-chloroanilino-, 4-p-chloroanilino-, 5-p-chloroanilino-. 6-p-chloroanilino-, 7-p-chloroanilino-, 6-(2,4-dichloro-anilino)-, 6-(2,5-dichloro-anilino)-, 6-(3,4-dichloro-anilino)-, 4-p-fluoroanilino-, 5-p-fluoroanilino-, 6-p-fluoroanilino-, 7-p-fluoroanilino-, 4-p-iodoanilino-, 5-p-iodoanilino-, 6-p-iodoanilino-, 7-p-iodoanilino-, 6-o-nitroanilino-, 6-m-nitroanilino-, 6-p-nitroanilino-, 6-o-aminoanilino-, 6-m-aminoanilino-, 6-p-aminoanilino-, 6-o-hydroxyanilino-, 6-m-hydroxyanilino-, 6-p-hydroxyanilino-, 6-o-sulfo-anilino-, 6-m-sulfo-anilino-, 6-p-sulfo-anilino-, 6-cyclohexylamino-, 4-benzyloxy-, 5-benzyloxy-, 6-benzyloxy-, 7-benzyloxy-, 6-o-toluidino-, 6-m-toluidino-, 6-p-toluidino-, 6-o-methoxyanilino-, 6-m-methoxyanilino-, 6-p-methoxyanilino-, 6-N-methylanilino-, 6-N-methyl-p-chloroanilino-, 4-benzylamino-, 5-benzylamino-, 6-benzylamino-, 7-benzylamino-, 6-o-carboxyanilino-, 6-m-carboxyanilino-, 6-p-carboxyanilino-, 6-benzylthio-, 6-p-chlorobenzylthio-, 6-N-ethylanilino-, 6-N-ethyl-p-chloroanilino-, 6-(2,5-dimethylanilino)-, 6-(2,4,6-trimethylanilino)-, 6-(1-naphthylamino)-, 6-(2-naphthylamino)-, 6-diphenylamino-, 6-p-phenylanilino-, 4-bromo-5-methyl-, 4-bromo-6-methyl-, 4-bromo-7-methyl-, 5-bromo-4-methyl-, 5-bromo-6-methyl-, 5-bromo-7-methyl-, 6-bromo-4-methyl-, 6-bromo-5-methyl-, 6-bromo-7-methyl-, 7-bromo-4-methyl-, 7-bromo-5-methyl-, 7-bromo-6-methyl-, 4-bromo-5-methoxy-, 4-bromo-6-methoxy-, 4-bromo-7-methoxy-, 5-bromo-4-methoxy-, 5-bromo-6-methoxy-, 5-bromo-7-methoxy-, 6-bromo-4-methoxy-, 6-bromo-5-methoxy-, 6-bromo-7-methoxy-, 7-bromo-4-methoxy-, 7-bromo-5-methoxy-, 7-bromo-6-methoxy-, 4,5-dichloro-, 4,6-dichloro-, 4,7-dichloro-, 5,6-dichloro-, 5,7-dichloro-, 6,7-dichloro-, 4-chloro-5-fluoro-, 4-chloro-6-fluoro-, 4-chloro-7-fluoro-, 5-chloro-4-fluoro-, 5-chloro-6-fluoro-, 5-chloro-7-fluoro-, 6-chloro-4-fluoro-, 6-chloro-5-fluoro-, 6-chloro-7-fluoro-, 7-chloro-4-fluoro-, 7-chloro-5-fluoro-, 7-chloro-6-fluoro-, 6-fluoro-4-methyl-, 4-chloro-5-methyl-, 4-chloro-6-methyl-, 4-chloro-7-methyl-, 5-chloro-4-methyl-, 5-chloro-6-methyl-, 5-chloro-7-methyl-, 6-chloro-4-methyl-, 6-chloro-5-methyl-, 6-chloro-7-methyl-, 7-chloro-4-methyl-, 7-chloro-5-methyl-, 7-chloro-6-methyl-, 6-bromo-4-methyl-, 6-iodo-4-methyl-, 4-chloro-5-methoxy-, 4-chloro-6-methoxy-, 4-chloro-7-methoxy-, 5-chloro-4-methoxy-, 5-chloro-6-methoxy-, 5-chloro-7-methoxy-, 6-chloro-4-methoxy-,

6-chloro-5-methoxy-, 6-chloro-7-methoxy-, 7-chloro-4-methoxy-, 7-chloro-5-methoxy-, 7-chloro-6-methoxy-, 4-methyl-6-nitro-, 4-methoxy-5-nitro-, 4-methoxy-6-nitro-, 4-methoxy-7-nitro-, 5-methoxy-4-nitro-, 5-methoxy-6-nitro-, 5-methoxy-7-nitro-, 6-methoxy-4-nitro-, 6-methoxy-5-nitro-, 6-methoxy-7-nitro-, 7-methoxy-4-nitro-, 7-methoxy-5-nitro-, 7-methoxy-6-nitro-, 6-amino-4-methyl-, 6-amino-5-methyl-, 6-amino-7-methyl-, 6-amino-4-methoxy-, 6-amino-5-methoxy-, 6-amino-7-methoxy-, 4-chloro-5-trifluoromethyl-, 4-chloro-6-trifluoromethyl-, 4-chloro-7-trifluoromethyl-, 5-chloro-4-trifluoromethyl-, 5-chloro-6-trifluoromethyl-, 5-chloro-7-trifluoromethyl-, 6-chloro-4-trifluoromethyl-, 6-chloro-5-trifluoromethyl-, 6-chloro-7-trifluoromethyl-, 7-chloro-4-trifluoromethyl-, 7-chloro-5-trifluoromethyl-, 7-chloro-6-trifluoromethyl-, 4,5-dimethyl-, 4,6-dimethyl-, 4,7-dimethyl-, 5,6-dimethyl-, 5,7-dimethyl-, 6,7-dimethyl-, 4,5-dimethoxy-, 4,6-dimethoxy-, 4,7-dimethoxy-, 5,6-dimethoxy-, 5,7-dimethoxy-, 6,7-dimethoxy-, 4-methyl-6-methoxy-, 4-methyl-6-trifluormethyl-, 4-methyl-6-methylthio-, 4-methoxy-6-methylthio-, 6-methoxy-4-methylthio-, 6-formamido-4-methyl-, 4-chloro-5-ethoxy-, 4-chloro-6-ethoxy-, 4-chloro-7-ethoxy-, 5-chloro-4-ethoxy-, 5-chloro-6-ethoxy-, 5-chloro-7-ethoxy-, 6-chloro-4-ethoxy-, 6-chloro-5-ethoxy-, 6-chloro-7-ethoxy-, 7-chloro-4-ethoxy-, 7-chloro-5-ethoxy-, 7-chloro-6-ethoxy-,

4-ethoxy-5-nitro-, 4-ethoxy-6-nitro-, 4-ethoxy-7-nitro-, 5-ethoxy-4-nitro-, 5-ethoxy-6-nitro-, 5-ethoxy-7-nitro-, 6-ethoxy-4-nitro-, 6-ethoxy-5-nitro-, 6-ethoxy-7-nitro-, 7-ethoxy-4-nitro-, 7-ethoxy-5-nitro-, 7-ethoxy-6-nitro-, 4-methyl-6-methylamino-, 4-methyl-6-ethyl-, 4-methyl-6-ethoxy-, 4-methyl-6-methoxycarbonyl-, 4-methyl-6-ethylamino-, 5-dimethylamino-4-methyl-, 6-dimethylamino-4-methyl-, 7-dimethylamino-4-methyl-, 6-acetamido-4-methyl-, 4-methyl-6-n-propylamino-, 6-isopropylamino-4-methyl-, 6-(1-carboxyethyl-amino)-4-methyl-, 6-diethylamino-4-methyl-, 4-methyl-6-(5-methyl-1,3,4-thiadiazol-2-yl-thio)-, 4-methyl-6-pyrrolidino-, 4-methyl-6-piperidino-, 4-methyl-6-(4-methyl-piperazino)-, 4-methyl-6-(N'-methylaza-pentamethylen)-hydrazino-, 4-methyl-6-(2-pyrimidylamino)-, 4-methyl-6-(4-pyridylthio)-, 4-methyl-6-(2-pyridylamino)-, 4-methyl-6-(3-pyridylamino)-, 4-methyl-6-(4-pyridylamino)-, 4-methyl-6-phenyl-, 6-o-chlorophenyl-4-methyl-, 6-m-chlorophenyl-4-methyl-, 6-p-chlorophenyl-4-methyl-, 4-methyl-6-phenoxy-, 6-o-chlorophenoxy-4-methyl-, 6-m-chlorophenoxy-4-methyl-, 6-p-chlorophenoxy-4-methyl-, 6-p-hydroxyphenoxy-4-methyl-, 6-(3,5-dihydroxyphenoxy)-4-methyl-, 4-methyl-6-phenylthio-, 6-p-chlorophenylthio-4-methyl-, 5-anilino-4-methyl-, 6-anilino-4-bromo-, 6-anilino-5-bromo-, 6-anilino-7-bromo-, 6-anilino-4-chloro-, 6-anilino-5-chloro-, 6-anilino-7-chloro-, 6-anilino-4-methyl-, 6-anilino-5-methyl-, 6-anilino-7-methyl-, 7-anilino-4-methyl-, 6-anilino-4-methoxy-, 6-anilino-5-methoxy-, 6-anilino-7-methoxy-, 7-anilino-6-methoxy-, 6-o-bromoanilino-4-methyl-, 6-m-bromoanilino-4-methyl-, 5-p-bromoanilino-4-methyl-, 6-p-bromoanilino-4-methyl-, 7-p-bromoanilino-4-methyl-, 6-o-chloroanilino-4-methyl-, 6-m-chloroanilino-4-methyl-, 5-p-chloroanilino-4-methyl-, 6-p-chloroanilino-4-methyl-, 6-p-chloroanilino-5-methyl-, 6-p-chloroanilino-7-methyl-, 7-p-chloroanilino-4-methyl-, 6-(2,4-dichloroanilino)-4-methyl-, 6-(2,5-dichloroanilino)-4-methyl-, 6-(3,4-dichloroanilino)-4-methyl-, 5-p-fluoro-anilino-4-methyl-, 6-p-fluoroanilino-4-methyl-, 7-p-fluoroanilino-4-methyl-, 5-p-iodoanilino-4-methyl-, 6-p-iodoanilino-4-methyl-, 7-p-iodoanilino-4-methyl-, 4-methyl-6-o-nitroanilino-, 4-methyl-6-m-nitroanilino-, 4-methyl-6-p-nitroanilino-, 6-o-aminoanilino-4-methyl-, 6-m-aminoanilino-4-methyl-, 6-p-aminoanilino-4-methyl-, 6-o-hydroxyanilino-4-methyl-, 6-m-hydroxyanilino-4-methyl-, 6-p-hydroxyanilino-4-methyl-, 4-methyl-6-o-sulfo-anilino-, 4-methyl-6-m-sulfo-anilino-, 4-methyl-6-p-sulfo-anilino-, 6-cyclohexylamino-4-methyl-, 6-p-chloroanilino-4-methoxy-, 6-p-chloroanilino-5-methoxy-, 6-p-chloroanilino-7-methoxy-, 4,6-di-tert.-butyl-, 6-benzyloxy-4-methyl-, 6-anilino-4-ethyl-, 6-p-chloroanilino-4-ethyl-, 6-anilino-4-ethoxy-, 6-p-chloroanilino-4-ethoxy-, 4-methyl-6-o-toluidino-, 4-methyl-6-m-toluidino-, 4-methyl-6-p-toluidino-, 6-o-methoxy-anilino-4-methyl-, 6-m-methoxyanilino-4-methyl-,

4-methyl-6-N-methyl-anilino-, 4-methyl-6-N-methyl-p-chloroanilino-, 4-methyl-6-benzylamino-, 6-o-carboxyanilino-4-methyl-, 6-m-carboxyanilino-4-methyl-, 6-p-carboxyanilino-4-methyl-, 6-benzylthio-4-methyl-, 6-p-chlorobenzylthio-4-methyl-, 6-N-ethylanilino-4-methyl-, 6-N-ethyl-p-chloro-anilino-4-methyl-, 4-methyl-6-(2,5-dimethylanilino)-, 4-methyl-6-(2,4,6-trimethylanilino)-, 4-methyl-6-(1-naphthylamino)-, 4-methyl-6-(2-naphthylamino)-, 6-diphenylamino-4-methyl-, 4-methyl-6-p-phenylanilino-, 4,5,6-trichloro-, 4,5,7-trichloro-, 4,6,7-trichloro-, 5,6,7-trichloro-, 4,5-dichloro-6-methyl-, 4,5-dichloro-7-methyl-, 4,6-dichloro-5-methyl-, 4,6-dichloro-7-methyl-, 4,7-dichloro-5-methyl-, 4,7-dichloro-6-methyl-, 5,6-dichloro-4-methyl-, 5,6-dichloro-7-methyl-, 5,7-dichloro-4-methyl-, 5,7-dichloro-6-methyl-, 6,7-dichloro-4-methyl-, 6,7-dichloro-5-methyl-, 4,5-dichloro-6-methoxy-, 4,5-dichloro-7-methoxy-, 4,6-dichloro-5-methoxy-, 4,6-dichloro-7-methoxy-, 4,7-dichloro-5-methoxy-, 4,7-dichloro-6-methoxy-, 5,6-dichloro-4-methoxy-, 5,6-dichloro-7-methoxy-, 5,7-dichloro-4-methoxy-, 5,7-dichloro-6-methoxy-, 6,7-dichloro-4-methoxy-, 6,7-dichloro-5-methoxy-, 4-chloro-5,6-dimethyl-, 4-chloro-5,7-dimethyl-, 4-chloro-6,7-dimethyl-, 5-chloro-4,6-dimethyl-, 5-chloro-4,7-dimethyl-, 5-chloro-6,7-dimethyl-, 6-chloro-4,5-dimethyl-, 6-chloro-4,7-dimethyl-, 6-chloro-5,7-dimethyl-, 7-chloro-4,5-dimethyl-, 7-chloro-4,6-dimethyl-, 7-chloro-5,6-dimethyl-, 4-chloro-5,6-dimethoxy-,

4-chloro-5,7-dimethoxy-, 4-chloro-6,7-dimethoxy-, 5-chloro-4,6-dimethoxy-, 5-chloro-4,7-dimethoxy-, 5-chloro-6,7-dimethoxy-, 6-chloro-4,5-dimethoxy-, 6-chloro-4,7-dimethoxy-, 6-chloro-5,7-dimethoxy-, 7-chloro-4,5-dimethoxy-, 7-chloro-4,6-dimethoxy-, 7-chloro-5,6-dimethoxy-, 4,5-dimethoxy-6-nitro-, 4,5-dimethoxy-7-nitro-, 4,6-dimethoxy-5-nitro-, 4,6-dimethoxy-7-nitro-, 4,7-dimethoxy-5-nitro-, 4,7-dimethoxy-6-nitro-, 5,6-dimethoxy-4-nitro-, 5,6-dimethoxy-7-nitro-, 5,7-dimethoxy-4-nitro-, 5,7-dimethoxy-6-nitro-, 6,7-dimethoxy-4-nitro-, 6,7-dimethoxy-5-nitro-, 6-chloro-7-isopropyl-4-methyl-, 6-anilino-4,5-dichloro-, 6-anilino-4,7-dichloro-, 6-anilino-5,7-dichloro-, 4,5-dichloro-6-p-chloroanilino-, 4,7-dichloro-6-p-chloroanilino-, 5,7-dichloro-6-p-chloroanilino-, 6-amino-5-chloro-4-methyl-, 6-anilino-5-chloro-7-methyl-, 5-chloro-6-p-chloroanilino-4-methyl-, 5-chloro-6-p-chloroanilino-7-methyl-, 6-anilino-7-chloro-4-methoxy-, 6-p-chloroanilino-7-chloro-4-methoxy-, 6-anilino-4,5-dimethyl-, 6-anilino-4,7-dimethyl-, 6-p-chloroanilino-4,5-dimethyl-, 6-p-chloroanilino-4,7-dimethyl-, 6-anilino-4,5-dimethoxy-, 6-anilino-4,7-dimethoxy-, 6-p-chloroanilino-4,5-dimethoxy-, 6-p-chloroanilino-4,7-dimethoxy-, 6-anilino-7-isopropyl-4-methyl-, 6-p-chloroanilino-7-isopropyl-4-methyl-1,3,2-benzothiazathiolium chloride,

furthermore, f.e., naphtho/2,1-d/-1,3,2-thiazathiolium-chloride (cf. The Ring Index, l.c., No. 2606 (1960)) naphtho/1,2-d/-1,3,2-thiazathiolium chloride, (cf. The Ring Index, l.c., No. 2607 (1960)), 5-chloronaphtho/1,2-d/-1,3,2-thiazathiolium chloride, carbazolo-/3,2-d/-1,3,2-thiazathiolium chloride (from 3-aminocarbazole; cf. Helv. Chim. Acta, vol. 15 (1932), page 290), 6-chloro-carbazolo/3,2-d/-1,3,2-thiazathiolium chloride and the corresponding bromides, hydroxides, perchlorates and tetrafluoroborates.

A particularly preferred compound is 6-p-chloroanilino-4-methyl-1,3,2-benzothiazathiolium chloride ("CMB"; Ia, $R^4$ = methyl, $R^5$ = $R^7$ = H, $R^6$ = p-chloro-phenylamino, X = Cl).

This substance can also be formulated as a derivative of E (see above):

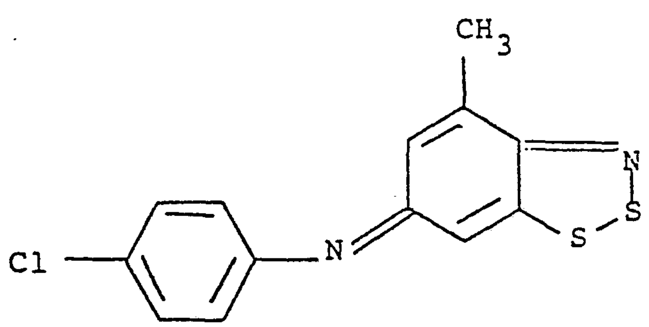

. HCl.

Some of the compounds of Formula I (particularly some of the compounds of Formula Ia) are known. Those which are novel can be prepared by methods which are in themselves known, e.g., as are described in the literature (for example, in the literature cited above), specifically under reaction conditions, e.g., as are known and are suitable for the reactions mentioned. In these methods, it is also possible to make use of variants which are in themselves known but are not mentioned here in greater detail.

The starting materials for the preparation of compounds of Formula I can, if desired, also be formed in situ, by not isolating them from the reaction mixture, but immediately reacting them further to give the compounds of Formula I.

The chlorides of Formula I (X = Cl) are preferably prepared by the reaction of an amine of Formula II

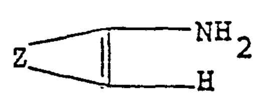
(II)

wherein Z is as above, or a reactive derivative of such amine, f.e. an acid addition salt (preferably the hydrochloride), N-acyl or N-alkyl derivative, with "sulfur monochloride" $S_2Cl_2$ ("Herz reaction"), usually in the presence or absence of an additional inert solvent such as formic acid, acetic acid, ligroin, benzene, toluene, xylene or nitrobenzene at temperatures between about 20 and 120°, preferably between 50 und 100°. A preferred method is the reaction of II-hydrochloride with an excess (molar ratio 3:1 or higher) of $S_2Cl_2$ without an additional solvent at 50 to 80°; under these conditions, the reaction needs about 1 to 4 hours.

Preferred amines II are aniline derivatives of Formula IIa

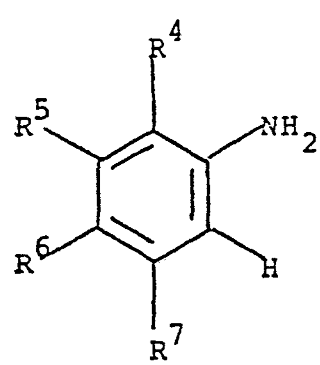

(IIa)

wherein

$R^4$ to $R^7$ are as above.

In detail, suitable amines of Formula II are f.e.: anilines containing at least one free o-position such as aniline, o-, m- and p-phenylene diamine, 2-, 3- and 4-bromo-aniline, 2-, 3- and 4-chloroaniline, 2-, 3- and 4-fluoroaniline, 2-, 3- and 4-iodoaniline, 2-, 3- and 4-nitroaniline, 2,3-, 2,4-, 2,5-, 3,4- and 3,5-dibromo-aniline, 2,3-, 2,4-, 2,5-, 3,4- and 3,5-dichloroaniline, 2,3-, 2,4-, 2,5-, 3,4- and 3,5-difluoroaniline, 2,4- and 3,5-dinitroaniline, 4-bromo-2-chloro-aniline, 2-bromo-4-nitro-aniline, 3-chloro-4-fluoro-aniline, 4-chloro-2-fluoro-aniline, 2-chloro-4- and -5-nitro-aniline, 4-chloro-2- and 3-nitro-aniline, 2,5-dichloro-4-nitro-aniline, 4,5-dichloro-2-nitro-aniline, 2-fluoro-4- and -5-nitro-aniline, 4-fluoro-2- and -3-nitro-aniline, 2,3,4-, 2,3,5-, 2,4,5- and 3,4,5-trichloro-aniline, 2,3,4,5-tetrachloro-aniline, 2-, 3- and 4-aminophenol, 2-amino-4-chlorophenol, 2-amino-4- and -5-nitro-phenol, 4-amino-2-nitro-phenol, 2-amino-4-chloro-5- and -6-nitro-phenol, 2-amino-6-chloro-4-nitro-phenol, 2-amino-4,6-dichloro-phenol, 4-amino-2,6-dichloro-phenol, 2-chloro-p-phenylene diamine, 4-chloro-o- and -m-phenylene-diamine, 2-nitro-p-phenylene-

diamine, 3- and 4-nitro-o-phenylenediamine, 4,5-dichloro-o-phenylenediamine, o-, m- and p-toluidine, o-, m- and p-anisidine, 4-methoxy-o- and -m-phenylenediamine, 2-methoxy-p-phenylenediamine, 4-methyl-m-phenylenediamine, 2-methyl-m- and -p-phenylenediamine, 4-methyl-o-phenylenediamine, 2-bromo-4-methylaniline, 4-bromo-2- and -3-methyl-aniline, 2-chloro-4-methyl-aniline, 3-, 4- and 5-chloro-2-methyl-aniline, 4-chloro-3-methyl-aniline, 3-chloro-4-methyl-aniline, 3,4-dichloro-2-methyl-aniline, 2-chloro-4- and -5-methoxy-aniline, 5-chloro-2-methoxy-4-nitro-aniline, 2-methyl-3-, -4- and -5-nitro-aniline, 4-methyl-2- and -3-nitro-aniline, 3-chloro-6-methyl-4-nitro-aniline, 5-chloro-2-methyl-4-nitro-aniline, 2-methoxy-4- and -5-nitro-aniline, 4-methoxy-2- and -3-nitro-aniline, 3-chloro-4-methoxy-aniline, 4-chloro-3-methoxy-aniline, 5-chloro-2-methoxy-aniline, 2-amino-4-methyl-, 4-amino-3-methyl-, 5-amino-2-methyl- and 2-amino-5-methyl-phenol, 2-amino-4-methyl-6-nitro-phenol, 5-fluoro-2-methyl-, 3-fluoro-4-methyl- and 4-fluoro-3-methyl-aniline, 2-, 3- and 4-aminobenzonitrile, 2-amino-4-chloro-, 2-amino-5-chloro- and 2-amino-4-nitro-benzonitrile, o-, m- and p-methylthio-aniline, o-, m- and p-trifluoromethyl-aniline, 2-chloro-5-, 3-chloro-5-, 4-chloro-2- and 4-chloro-3-trifluoromethyl-aniline, 2-nitro-4- and 4-nitro-2-trifluoromethyl-aniline, 2-, 3- and 4-ethylaniline, 2,3-, 2,4-, 2,5-, 3,4- and 3,5-dimethyl-aniline, 2-, 3- and 4-ethoxyaniline, 2-methoxy-5-methyl- and 4-methoxy-2-methyl-aniline, 2-, 3- and 4-amino-acetophenone, N,N-dimethyl-o-, -m- and -p-phenylenediamine, 4,5-dimethyl-o-phenylene-diamine, 2-, 3- and 4-amino-acetanilide, 3-amino-4-chloro-acetanilide, 2,3-, 2,4-, 2,5-, 3,4- and 3,5-dimethoxy-aniline, 5-chloro-2,4-dimethoxy-aniline, 3,4-dimethoxy-2-nitro-aniline, 4-ethoxy-2-nitro-aniline, 4-chloro-2,3-dimethyl- and 4-chloro-2,5-dimethyl-aniline, 2,6-dimethyl-3-nitro-, 4,5-dimethyl-2-nitro- and 3,4-dimethyl-6-nitro-aniline, 4-amino-2,5-dimethyl-phenol, 6-amino-2,4-dimethyl-phenol, 3,5-bis-trifluoromethyl-aniline, 4-amino-2,5-dimethoxy-benzonitrile,2-isopropenyl-aniline, 2-, 3- and 4-n-propylaniline, 2-, 3- and 4-iso-propyl-aniline, 4-cyclopropyl-aniline, 2-, 3- and 4-n-propyloxy-aniline, 2-, 3- and 4-isopropyloxy-aniline, 3,4,5-trimethoxyaniline, N-acetyl-N-methyl-1,4-phenylene-diamine, 3-amino-2-methyl-acetanilide, 2-ethoxy-5-methyl-aniline, 4- and 5-amino-indane, 3-amino-4-ethoxy-acetanilide, 2-, 3- and 4-n-butylaniline, 2-, 3- and 4- iso-butylaniline, 2-methyl-5-isopropyl-aniline, 2-, 3- and 4-n-butyloxy-aniline, 2-, 3- and 4-isobutyloxy-aniline, 2,5- and 3,4-diethoxyaniline, N,N-diethyl-1,4-phenylene-diamine; 2-amino-4-tert.-butyl-phenol; aminonaphthalenes

such as 1- and 2-naphthylamine, 1,5-, 1,8- and 2,3-diaminonaphthalene, 1-amino-7-naphthol, 2-, 3- and 4-aminodiphenyl, amino-halo-diphenyls such as 4-amino-4'-chlorodiphenyl, 2-, 3- and 4-amino-diphenylamine, diaminodiphenyls such as benzidine, 2,2'-dichloro-, 3,3'-dichloro- and 2,2',5,5'-tetrachloro-benzidine; diaminodiphenylamines such as 4,4'-diamino-diphenylamine; 4-aminoazobenzene; amino-diphenylethers such as 2-, 3- and 4-aminodiphenylether, 2-amino-4-chloro-, 4-amino-4'-chloro- and 2-amino-4,4'-dichloro-diphenylether; aminodiphenylsulfones such as 2-, 3- and 4-aminodiphenylsulfone, 4,4'-diaminodiphenylsulfone; aminobenzophenones such as 2- and 4-aminobenzophenone, 2-amino-4- and -5-chloro-benzophenone; N-benzoyl-1,4-phenylenediamine, 4,4'-diamino-diphenyl-methane, 2-amino-4-methyldiphenyl sulfide, N-(p-aminobenzoyl)-1,4-phenylenediamine, 3,3'-dimethoxybenzidine, 2,2'- and 3,3'dimethylbenzidine, N-monoacetyl-benzidine, 4-amino-3,2'-dimethyl- and 4-amino-2,5-dimethoxy-azobenzene, 2,5-di-n-butoxyaniline, 1- and 2-aminoanthraquinone, 1,4- and 1,5-diamino-anthraquinone, 2-amino-1-chloro-, 2-amino-3-chloro-, 1-amino-4-hydroxy-, 1,5-diamino-4,8-dihydroxy-, and 1,4-diamino-5-nitro-anthraquinone, bis-(3-methyl-4-aminophenyl)-methane, 4-benzoylamino-2,5-dimethoxy-aniline, 4-benzoylamino-2-methoxy-5-methyl-aniline, 4-benzoylamino-2,5-diethoxy-aniline,

1,1-bis-(4-aminophenyl)-cyclohexane, 1-amino-4- and -5-benzoylamino-anthraquinone,2- and 3-aminofuran, 2- and 3-aminothiophene, 2-, 3- and 4-amino-pyridine, 2,3- and 2,6-diaminopyridine, 2-amino-5-chloro-, 3-amino-2-chloro-, and 2-amino-5-nitro-pyridine, 2-amino-3-, -4- and -6-methyl-pyridine, 5-amino-2-methoxy-pyridine, 3,4-methylenedioxy-aniline, 3- and 8-amino-quinoline, 1-phenyl-3-methyl-5-amino-pyrazole, 2-amino-5,7-dimethyl-1,8-naphthyridine, 1-(3-aminophenyl)- and 1-(4-aminophenyl)-3-methyl-5-pyrazolone, 2,6-diamino-3-phenylazo-pyridine.

Suitable are also the acid addition salts of these bases, preferably the hydrochlorides, hydrobromides, sulfates or nitrates, furthermore N-acyl derivatives (wherein the acyl group has preferably 1 to 7 carbon atoms such as formyl, acetyl or benzoyl), f.e. acetanilide, N-formyl-o-,-m- and p-toluidine-N-acetyl-o-, -m- and -p-toluidine, N-benzoyl-o-, -m- and -p-toluidine, N-acetyl-1- and -2-naphthylamine, and N-alkyl derivatives (wherein the alkyl group has preferably 1 to 4 carbon atoms such as methyl, ethyl or butyl), f.e. N-methyl-aniline, N-methyl-o-, -m- or -p-toluidine, N-ethyl-o-, -m- or -p-toluidine.

It is well known that substitution in the p-position of the benzene nucleus occurs during a Herz reaction, and if this position is already occupied, replacement by a chlorine atom may occur. Thus aniline yields 6-chloro-1,3,2-benzothiazathiolium chloride, and o-toluidine yields 6-chloro-4-methyl-1,3,2-benzothiazathiolium chloride.

A chlorine atom (or an alkoxygroup) in 6-position of the benzothiazathiolium salt is easily replaced by nucleophilic reagents, in particular amines, and compounds of Formula Ia, wherein $R^6$ is an amino or a mono- or disubstituted amino group, are advantageously obtainable by reaction of 6-chloro or 6-alkoxy compounds of Formula Ia ($R^6$ = Cl) with the appropriate amine. This reaction is preferably performed in the presence of an inert solvent such as acetic acid at temperatures between about 0 and 50°, especially between 15 and 30°. A base can be added in order to neutralize the hydrogen chloride formed; it is usual, however, to apply an excess of the amine for this purpose.

Suitable amines are those of Formula II, preferably the aniline derivatives, particularly those of formula IIa mentioned above, such as aniline, o-, m- and p-chloro-aniline, o-, m- and p-aminophenol and so on. In addition, 2,6-disubstituted anilines such as

2,6-difluoro-, 2,6-dichloro-, 2,6-dibromo-, 2,3,6- or 2,4,6-trifluoro-, 2,3,4,6-tetrafluoro-,pentafluoro-, 2,3,6- or 2,4,6-trichloro-, 2,3,4,6-tetrachloro-, pentachloro-, 2,6-dihydroxy-, 2,6-dimethyl-, 2,4,6-trimethyl- or 2,6-dimethoxy-aniline, are suitable, also other primary and secondary amines, f.e. mono-alkylamines of preferably 1 to 8 carbon atoms such as methyl-, ethyl-, n-propyl-, isopropyl-, butyl-, isobutyl-, sec.-butyl-, tert.-butyl-, n-pentyl-, n-hexyl-, n-heptyl-, n-octylamine; dialkylamines of preferably 1 to 8 carbon atoms in each alkyl group such as dimethyl-, N-methyl-N-ethyl-, diethyl-, di-n-propyl-, diisopropyl-, di-n-butyl-, diisobutyl-, di-sec.-butyl-, dipentyl-, dihexyl-, dioctylamine; substituted alkylamines and dialkylamines of preferably 1 to 12 carbon atoms, f.e. hydroxyamines such as ethanolamine,diethanolamine, 1-amino-2-propanol, 3-amino-1-propanol, 2-methylamino-ethanol, 2-amino-1-butanol, 2-ethylamino-ethanol, diisopropanolamine, 2-butylamino-ethanol, 2-benzylamino-ethanol; di- and polyamines such as ethylene diamine, 1,2- and 1,3-diaminopropane, 2-dimethylamino-ethylamine, 3-methylamino-propylamine, 2-ethylamino-ethylamine, 3-dimethylamino-propylamine, 2-diethylamino-ethylamine, 3-diethylamino-propylamine, ether-amines such as 2-methoxy-ethylamine, 2-ethoxy-ethylamine, 3-methoxy-propylamine, 3-ethoxy-propylamine,

2-propoxy-ethylamine, bis-2-methoxyethyl-amine; aminoacetals such as aminoacetaldehyde-dimethyl- or diethylacetal; alkenylamines of preferably 2 to 8 carbon atoms such as allylamine, diallylamine; alkinylamines of preferably 2 to 8 carbon atoms such as propargyl-amine; cycloalkylamines of preferably 3 to 10 carbon atoms such as cyclopropyl-, cyclobutyl-, cyclopentyl-, cyclohexyl-, cycloheptyl-, 2-, 3- or 4-methylcyclohexyl-, N-methyl-cyclohexyl-, cyclooctyl-, N-ethyl-cyclohexyl-, N-propyl-cyclohexyl-, N-isopropyl-cyclohexyl-, N-butyl-cyclohexyl-, N-isobutyl-cyclohexyl-, N-sec.-butyl-cyclo-hexyl-, 4-tert.-butyl-cyclohexylamine; saturated cyclic amines of preferably 4 to 8 carbon atoms such as pyrrolidine, piperidine, morpholine, piperazine, 4-alkyl-piperazines (wherein the alkyl group is preferably of 1 to 4 carbon atoms), f.e. 4-methyl-piperazine; N-alkyl-N-arylamines of preferably 7 to 11 carbon atoms such as N-methyl-aniline, N-ethyl-aniline; substituted N-alkyl-N-arylamines of preferably 7 to 11 carbon atoms such as N-methyl-o-, -m- or -p-chloroaniline, N-ethyl-o-, -m- or -p-chloroaniline; diarylamines of preferably 12 to 18 carbon atoms such as diphenylamine; arylalkylamines of preferably 7 to 11 carbon atoms such as benzylamine, 1- or 2-phenylethylamine, 1- or 2-naphthylmethylamine, N-benzyl-N-methylamine,

N-benzyl-N-ethylamine; substituted arylalkylamines of preferably 7 to 11 carbon atoms such as o-, m- or p-fluorobenzylamine, o-, m- or p-chlorobenzylamine.

The compounds of Formula I (particularly Formula Ia) can also be obtained by reaction of corresponding 2-amino-thiols of Formula III

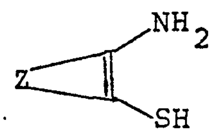 (III)

wherein Z is as above, with thionyl chloride, in the presence or (preferably) in the absence of an additional inert solvent, preferably at temperatures between 0° and 80°. In particular, those compounds of Formula Ia having hydrogen at the 6-position can be obtained by this method.

Hydroxides of Formulae I or Ia (X = OH) can be prepared by hydrolysis of the corresponding salts, particularly the chlorides (X = Cl), f.e. by stirring with water at temperatures between 15 and 30°.

A hydroxide of Formula I (X = OH) can be converted into an appropriate salt of Formula I ($X^{\ominus}$ = anion of a strong acid) by treatment with such strong acid. Acids which give physiologically acceptable salts are suitable for this reaction. Thus, it is possible to use inorganic acids, for example sulfuric

acid, hydrogen halide acids, e.g., hydrochloric acid or hydrobromic acid, phosphoric acids, e.g. orthophosphoric acid, nitric acid or sulfamic acid, and also strong organic acids, specifically, for example,, aliphatic, alicyclic, araliphatic, aromatic or heterocyclic mono-basic or polybasic carboxylic, sulfonic or sulfuric acids, e.g., formic acid, methanesulfonic or ethanesulfonic acid, ethanedisulfonic acid, 2-hydroxyethanesulfonic acid, benzenesulfonic acid, p-toluenesulfonic acid, naphthalenemonosulfonic and naphthalenedisulfonic acids and laurylsulfuric acid.

It has been found that the compounds of Formula I have valuable pharmacological properties and are well tolerated. Thus, for example, inhibiting effects on the binding of rat myeloma immunoglobulin E to receptors on rat peritoneal mast cells are observed.

Furthermore, activities in inhibiting the IgE mediated release of histamine from human basophils and rat mast cells are noted.

Compounds of Formula I have been found active in preventing mast cells in the skin of rats from being passively sensitized by antisera. These pharmacological activities were determined by conventional tests: For human basophil see Siraganian, R.P. and Brodsky, M.J., Automated Histamine Analysis for In Vitro Testing, J. Allergy Clin. Immunol. 57, 525-540 (1976), and

Siraganian, R.P., Histamine Release and Assay Methods for the Study of Human Allergy, Manual of Clinical Immunology, Rose, N.R. and Friedman, H., Editors, Washington, American Society for Microbiology, 1976 Chapter 80, p.603. For IgE binding see Coutts, Stephen M., Reid, Dorothy M., Weinryb, Ira, Facile _In Vitro_ Method for Screening Inhibitors of IgE Binding to Mast Cells, J. of Pharmaceutical Sciences, Vol. 68, No. 3, March 1979, pp. 353-357. For mast cell histamine release see Coutts, Stephen M., Nehring, Jr., Robert E., Jariwala, Navin U., Purification of Rat Peritoneal Mast Cells: Occupation of IgE-Receptors by IgE Prevents Loss of the Receptors, The Journal of Immunology, Vol. 124, No. 5, May 1980, pp. 2309-2315.

The compounds of Formula I can, therefore, be used as active ingredients in medicaments in human and veterinary medicine for administration, e.g., to mammals including humans, and also as intermediate products for the preparation of other active compounds for medicaments.

Thus, this invention, in another aspect, also relates to the use of the compounds of Formula I in preparation of pharmaceutical formulations, especially by a non-chemical route. In such methods, they can be brought into a suitable dosage form together with at least one excipient or auxiliary and, if appropriate, in combination with one or more additional active compounds.

The invention, in another aspect also relates to agents, in particular pharmaceutical formulations, containing at least one compound of Formula I. These formulations can be employed as medicaments in human or veterinary medicine. Suitable excipients include organic or inorganic substances which are suitable for enteral (for example oral) or parenteral administration or for topical application and which do not react with the active compounds, for example, water, vegetable oils, benzyl alcohols, polyethylene glycols, gelatin, carbohydrates, such as lactose or starch, magnesium stearate, talc or petroleum jelly.

Tablets, dragees, capsules, syrups, elixirs, drops or suppositories are used particularly for enteral administration, solutions, preferably oil-based or aqueous solutions, and also suspensions, emulsions or implants are used particularly for parenteral administration, while ointments, creams or powders are used for topical application. The compounds can also be lyophilized and the resulting lyophilizates can be used, for example, for the preparation of injection formulations. Furthermore, the compounds can be applied in the form of inhalation capsules, inhalation solutions, nasal sprays, nasal spray powders or eye drops. The formulations indicated can be sterilized and/or can contain auxiliaries, such as lubricants, preservatives, stabilizers and/or wetting agents, emulsifiers, salts for influencing the osmotic pressure, buffer substances, coloring substances, flavoring substances and/or aroma generating substances. If desired, they can also contain one or more additional active compounds, for example, one or more vitamins.

The present invention, in another aspect, also relates to the use of the compounds of Formula I in therapeutic treatment of the human or animal body and in combating diseases, in particular all forms

of allergic and asthmatic diseases, specifically asthma bronchiale, allergic bronchitis, asthmatic bronchitis, asthma with infectious-allergic background, food allergy (cases of eczema, urticaria, Quincke edema, pruritus, gastrointestinal disturbances, asthma and rhinitis caused by food allergens), hay fever, allergic rhinitis (seasonal and perennial), allergic conjunctivitis, allergic unspecific irritation of the conjunctiva. In general, the substances of this invention are administered in analogy to known, commercially available formulations with a similar indication (for example disodium cromoglycate (cromolyn) preferably in dosages of approximately 1 - 400 mg, in particular, of 5 -200 mg, per dosage unit. In case the compounds are used in solution, 1 ml of the solution usually contains 1 to 400, preferably 5 to 200 mg. The daily dosage is preferably approximately 0.02-5 mg/kg of body weight. The particular dose for each specific patient as usual depends, however, on very diverse factors, for example, on the activity of the particular compound employed, on the age, body weight, general condition of health, sex, diet, etc. of the patient, on the time and route of administration, on the rate of excretion, on the combination of medicaments and on the severity of the particular disease to which the therapy relates. Oral administration is preferred.

Preferred tablets comprise a carbohydrate such as lactose or starch. They may contain a lubricant such as calcium stearate or magnesium stearate and/or talc. Each tablet preferably contains 1 - 400 mg, in particular 5-50 mg of the active compound.

Preferred dragees comprise a carbohydrate such as lactose or starch. They may contain a lubricant such as calcium stearate or magnesium stearate and/or talc. The coating preferably comprises a sweetening agent, e.g., a sugar such as sucrose. Each dragee preferably contains 1-400 mg, in particular 5-200 mg of the active compound.

Preferred capsules comprise hard or soft gelatine. Each capsule preferably contains 1-400 mg, in particular 5-200 mg of the active compound.

Preferred syrups comprise a sweetening agent, e.g., a sugar such as sucrose. A unit dose (5 ml) of the syrup preferably contains 1-400 mg, in particular 5-200 mg of the active compound.

Preferred drops, e.g., eye drops, comprise sterile water. The preferred concentration of the active compounds in these drops is about 1 - 400 mg, preferably 5 to 200 mg, per ml.

Preferred suppositories comprise polyethylene glycol or cocoa butter. Each suppository preferably contains 1-400 mg, in particular 5-200 mg of the active compound.

Preferred injection or inhalation solutions, suspensions or emulsions comprise sterile water. Solutions preferably are filled into ampoules; if desired, they can be lyophilized. Emulsions contain one or more emulsifiers, in addition. A unit dose (e.g., 1 ml) of the solution, suspension or emulsion preferably contains 1-400 mg, in particular 5 -200 mg of the active compound.

Preferred sprays contain water, an alcohol of 1 to 4 carbon atoms and/or one or more halogenated hydrocarbon(s). The preferred concentration of the active compounds in these sprays is about 1 to 400 mg, preferably 5 to 200 mg, per ml.

Preferred ointments, salves and creams contain one or more viscous to semi-solid or solid excipients, indigenous to topical application and having a dynamic viscosity preferably greater than water, e.g., a polyethylene glycol, petroleum jelly and/or a fat. Creams, in addition, contain water. The preferred concentration of the active compounds in these ointments, salves and creams is about 1 to 200 mg, preferably 5 to 50 mg, per ml.

It is also to be noted that compounds of Formula I, particularly those of Formula Ia, can be used as post-emergent herbicides and dyestuff intermediates in the same manner as the known compounds of the prior art.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. In the following examples, all temperatures are set forth uncorrected in degrees Celsius; unless otherwise indicated, all parts and percentages are by weight. Rf values refer to thin layer chromatograms on silica gel with dioxane-water 9:1 unless noted otherwise.

EXAMPLE 1

a) Under a nitrogen atmosphere, a suspension of 14.4 g of o-toluidine hydrochloride in 51 ml of disulfur dichloride is stirred for 1.5 hours at 60°. After adding 500 ml of toluene, 22.6 g of 6-chloro-4-methyl-1,3,2-benzothiazathiolium chloride is filtered off.

b) With stirring, 10 g of 6 chloro-4-methyl-1,3,2-benzothiazathiolium chloride are suspended in 100 ml of water. After 1.5 hours stirring at 20°, the mixture is filtered, the crude product is dried, dissolved in a small amount of methanol and purified chromatographically using ether/petroleum ether on silica gel; 6-chloro-4-methyl-1,3,2-benzothiazathiolium hydroxide (tautomeric structure: 6-chloro-4-methyl-3H-1,2,3-benzodithiazole-2-oxide) is obtained; m.p. 123° (dec.); Rf. 0.65 (ethyl acetate).

EXAMPLE 2

a) 22.6 g of 6-chloro-4-methyl-1,3,2-benzothiazathiolium chloride and 25.9 g of p-chloroaniline are dissolved in 250 ml of acetic acid at room temperature. After standing over night, the precipitated 6-p-chloroanilino-4-methyl-1,3,2-benzothiazathiolium chloride ("CMB") is filtered off and recrystallized from acetic acid (tautomeric structures: 6-p-chlorophenylimino-4-methyl-6H-1,2,3-benzodithiazole hydrochloride or 6H-1,2,3-benzodithiazole-6-p-chlorophenyl-iminium chloride).

M.p. 190 - 192°; decomposition at 200°. Infrared spectrum (in KBr): 1610, 1590, 1490, 1460, 1375, 1310, 1290, 775 $cm^{-1}$. Rf 0.38 (benzene/ethyl acetate 7:3). Mass spectrum m/e (relative intensity in %): 294 (42), 293 (26), 292 (100), 277 (13), 257 (23), 242 (6), 224 (4), 193 (7), 167 (6), 154 (10), 111 (11), 75 (13), 36 (7).

b) In a separatory funnel, 1 g of CMB is suspended in 400 ml of water. An equimolar amount of 1 N sodium hydroxide solution is added portionwise. After each addition, the mixture is extracted with ethyl acetate. The combined extracts are concentrated. 6-p-chlorophenylimino-4-methyl-6H-1,2,3-benzodithiazole (Formula G, $R^1$ = p-chlorophenyl, $R^4$ = methyl, $R^5$ = $R^7$ = H) is obtained and filtered off.

Infrared spectrum (in KBr): 1610, 1520, 1480, 1450, 1440, 1310, 1200, 755 $cm^{-1}$.

EXAMPLE 3

In analogy to Example 2, but using p-aminophenol instead of p-chloroaniline, 6-p-hydroxyanilino-4-methyl-1,3,2-benzothiazathiolium chloride is obtained, m.p. 292° (decomposition); infrared spectrum (in KBr): 1610, 1500, 1470, 1390, 1270, 1220, 1170, 825 $cm^{-1}$; Rf 0.48.

EXAMPLES 4 to 55

In analogy to Example 2, the following 4-methyl-1,3,2-benzothiazathiolium chlorides are obtained from 6-chloro-4-methyl- 1,3,2-benzothiazathiolium chloride and the corresponding amines:

4. 6-Anilino-, Rf 0.73,
5. 6-o-Fluoroanilino-,
6. 6-m-Fluoroanilino-,
7. 6-p-Fluoroanilino-, Rf 0.84,
8. 6-o-Chloroanilino-, Rf 0.74.

9. 6-m-Chloroanilino-, Rf 0.71.

10. 6-o-Bromoanilino-.

11. 6-m-Bromoanilino-.

12. 6-p-Bromoanilino-, Rf 0.72.

13. 6-o-Iodoanilino-.

14. 6-m-Iodoanilino-.

15. 6-p-Iodoanilino-, Rf 0.80.

16. 6-o-Nitroanilino-.

17. 6-m-Nitroanilino-.

18. 6-p-Nitroanilino-, Rf 0.74.

19. 6-o-Aminoanilino-, Rf 0.73.

20. 6-m-Aminoanilino-.

21. 6-p-Aminoanilino-, Rf 0.37 (butanol/acetic acid/water 3:1:1).

22. 6-o-Hydroxyanilino-.

23. 6-m-Hydroxyanilino-.

24. 6-o-Sulfoanilino-.

25. 6-m-Sulfoanilino-,

26. 6-p-Sulfoanilino-.

27. 6-o-Toluidino-.

28. 6-m-Toluidino-.

29. 6-p-Toluidino-.

30. 6-o-Methoxyanilino-.

31. 6-m-Methoxyanilino-.

32. 6-p-Methoxyanilino-, Rf 0.75.

33. 6-o-Trifluoromethylanilino-.

34. 6-m-Trifluoromethylanilino-.

35. 6-p-Trifluoromethylanilino-.

36. 6-o-Carboxyanilino-.

37. 6-m-Carboxyanilino-.

38. 6-p-Carboxyanilino-, Rf 0.70.

39. 6-p-Methylthioanilino-.

40. 6-p-Methylaminoanilino-.

41. 6-p-Ethylanilino-.

42. 6-p-Dimethylaminoanilino-.

43. 6-p-Phenylanilino-; free base (= 6-p-biphenylyl-imino-4-methyl-6H-1,2,3-benzodithiazole), Rf 0.73.

44. 6-(2,3-Dichloroanilino)-.

45. 6-(2,4-Dichloroanilino)-, Rf 0.78.

46. 6-(2,5-Dichloroanilino)-.

47. 6-(2,6-Dichloroanilino)-.

48. 6-(3,4-Dichloroanilino)-, Rf 0.77.

49. 6-(3,5-Dichloroanilino)-.

50. 6-(N-Methylanilino)-, Rf 0.37 (butanol/acetic acid/water 3:1:1).

51. 6-(p-Chloro-N-methylanilino)-,

52. 6-(2,4,6-Trimethylanilino)-, free base ( = 4-methyl-6-(2,4,6-trimethylphenylimino-6H-1,2,3-benzodithiazole), Rf 0.77.

53. 6-Benzylamino-.

54. 6-p-Chlorobenzylamino-.

55. 6-Diphenylamino-.

56. 6-p-Formamidoanilino-, Rf 0.64.

57. 6-p-Aminosulfonylanilino-; free base (= 6-p-amino-sulfonyl-phenylimino-4-methyl-6H-1,2,3-benzodithiazole), Rf 0.73.

58. 6-(3,4,5-Trimethoxyanilino)-; free base ( = 4-methyl-6-(3,4,5-trimethoxyphenylimino)-6H-1,2,3-benzodithiazole), Rf 0.70.

59. 6-m-Anilinocarbonylanilino-, Rf 0.75.

60. 6-(2,6-Dimethylanilino)-, Rf 0.74.

61. 6-p-Benzamidoanilino-, Rf 0.72.

62. 6-p-(p-Hydroxyphenylacetamido)-anilino-, Rf 0.68.

63. 6-p-Phenylazoanilino-, Rf 0.80.

64. 6-(3,4-Methylenedioxyanilino)-, Rf 0.75.

65. 6-(1-Naphthylamino)-; free base ( = 4-methyl-6-(1-naphthyl-imino)-6H-1,2,3-benzodithiazole), Rf 0.76.

66. 6-(1H-Indazol-6-ylamino)-, Rf 0.67.

67. 6-(2,4-Dimethoxyanilino)-.

68. 6-(p-Hydroxy-N-methylanilino)-.

EXAMPLES 69 to 126

In analogy to Example 2, the following 1,3,2-benzothiazathiolium chlorides are obtained from the corresponding 6-chloro-1,3,2-benzothiazathiolium chlorides and the corresponding amines:

69. 6-Anilino-, Rf 0.28 (toluene/ethyl acetate 7:3).

70. 6-p-Fluoroanilino-.

71. 6-o-Chloroanilino-.

72. 6-m-Chloroanilino-, Rf 0.32 (toluene/ethyl acetate 7:3).

73. 6-p-Chloroanilino-, Rf 0.70.

74. 6-p-Bromoanilino-.

75. 6-p-Iodoanilino-.

76. 6-p-Nitroanilino-.

77. 6-p-Aminoanilino-, Rf 0.66.

78. 6-p-Hydroxyanilino-, Rf 0.70.

79. 6-p-Sulfoanilino-.

80. 6-p-Toluidino-.

81. 6-p-Methoxyanilino-.

82. 6-p-Trifluoromethylanilino-.

83. 6-p-Carboxyanilino-.

84. 6-(2,4-Dichloroanilino)-.

85. 6-(3,4-Dichloroanilino)-.

86. 6-(N-Methylanilino)-.

87. 6-Anilino-4-chloro-.

88. 4-Chloro-6-p-fluoroanilino-.

89. 4-Chloro-6-o-chloroanilino-.

90. 4-Chloro-6-m-chloroanilino-.

91. 4-Chloro-6-p-chloroanilino-, Rf 0.49 (toluene/ethyl acetate 7:3).

92. 6-p-Bromoanilino-4-chloro-.

93. 4-Chloro-6-p-iodoanilino-.

94. 4-Chloro-6-p-nitroanilino-.

95. 6-p-Aminoanilino-4-chloro-, Rf 0.27 (butanol/acetic acid/water 3:1:1)

96. 4-Chloro-6-p-hydroxyanilino-, Rf 0.75.

97. 4-Chloro-6-p-sulfoanilino-.

98. 4-Chloro-6-p-toluidino-.

99. 4-Chloro-6-p-methoxyanilino-.

100. 4-Chloro-6-p-trifluoromethylanilino-.

101. 6-p-Carboxyanilino-4-chloro-.

102. 4-Chloro-6-(2,4-dichloroanilino)-.

103. 4-Chloro-6-(3,4-dichloroanilino)-.

104. 4-Chloro-6-(N-methylanilino)-.

105. 6-Anilino-4-methoxy-.

106. 6-p-Fluoroanilino-4-methoxy-.

107. 6-o-Chloroanilino-4-methoxy-.

108. 6-m-Chloroanilino-4-methoxy-.

109. 6-p-Chloroanilino-4-methoxy-, Rf 0.73.

110. 6-p-Bromoanilino-4-methoxy-.

111. 6-p-Iodoanilino-4-methoxy-.

112. 4-Methoxy-6-p-nitroanilino-.

113. 6-p-Aminoanilino-4-methoxy-, Rf 0.40 (butanol/ acetic acid/ water 3:1:1).

114. 6-p-Hydroxyanilino-4-methoxy-, Rf 0.66.

115. 4-Methoxy-6-p-sulfoanilino-.

116. 4-Methoxy-6-p-toluidino-.

117. 4-Methoxy-6-p-methoxyanilino-.

118. 4-Methoxy-6-p-trifluoromethylanilino-.

119. 6-p-Carboxyanilino-4-methoxy-.

120. 6-(2,4-Dichloroanilino)-4-methoxy-.

121. 6-(3,4-Dichloroanilino)-4-methoxy-.

122. 4-Methoxy-6-(N-methylanilino)-.

123. 5-Chloro-6-p-chloroanilino-, Rf 0.83.

124. 5-Chloro-6-p-hydroxyanilino-, Rf 0.79.

125. 4-Benzyloxy-6-p-chloroanilino-, Rf 0.77.

126. 6-p-Chloroanilino-4-hydroxy-,

EXAMPLE 127

Analogously to Example 2, 6-methoxy-4-methyl-1,3,2-benzothiazathiolium chloride is reacted with p-chloroaniline to yield CMB,m.p. 190 - 192$^{o}$ (decomposition at 200$^{o}$).

EXAMPLES 128 to 251

Analogously to Example 127, reaction of the corresponding 6-methoxy-1,3,2-benzothiazathiolium chlorides with the corresponding amines yields the compounds described in Examples 3 to 126.

EXAMPLE 252

In analogy to Example 1a, 26,9 g of 4-amino-4'-chloro-2-methyl-diphenylamine-hydrochloride is reacted with 80 ml of $S_2Cl_2$ to yield CMB, m.p. 190 - 192$^{o}$ (decomposition at 200$^{o}$).

EXAMPLE 253

A total of 100 ml of thionyl chloride is added to 26,5 g of 4-amino-4'-chloro-3-mercapto-5-methyl-diphenylamine (Rf 0,48; benzene/ethyl acetate 7:3). The mixture is refluxed for 0.5 hours, mixed with benzene and filtered. The precipitated CMB is washed with benzene and dried. M.p. 190 - 192° (decomposition at 200°).

The following examples relate to pharmaceutical formulations containing compounds of Formula I:

EXAMPLE A: Tablets

A mixture of 1 kg of CMB, 4 kg of lactose, 1.2 kg of maize starch, 0.2 kg of talc and 0.1 kg of magnesium stearate is pressed in the customary manner to give tablets, in such a way that each tablet contains 10 mg of active compound.

EXAMLE B: Dragees

Tablets are pressed analogously to Example A and are subsequently coated in a customary manner with a coating consisting of sucrose, wheat starch, talc, tragacanth and colorant.

EXAMPLE C: Inhalation capsules

5 kg of CMB and 5 kg of lactose are filled into capsules in a customary manner, so that each capsule contains 20 mg of the active compound. The capsules may be applied by means of an inhalator.

EXAMPLE D: Inhalation solution

1 kg of CMB is dissolved in 100 l of sterile distilled water. The solution is filtered and filled into ampoules. Each ampoule (1 ml) contains 10 mg of active substance.

Tablets, dragees, capsules and inhalation solutions containing one or more of the remaining active compounds of Formula I can be obtained analogously.

The preceding examples can be repeated with similar success by substituting the generically or specifically described reactants and/or operating conditions of this invention for those used in the preceding examples.

From the foregoing description, one skilled in the art can easily ascertain the essential characteristics of this invention, and without departing from the spirit and scope thereof, can make various changes and modifications of the invention to adapt it to various usages and conditions.

WHAT IS CLAIMED IS:

1. A pharmaceutical composition comprising a compound of Formula I

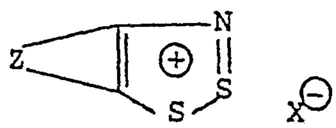

wherein 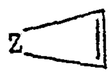 stands for an aromatic or heteroaromatic system which may be substituted or unsubstituted and

$X^{\ominus}$ is $OH^{\ominus}$ or an anion of a strong acid.

2. A pharmaceutical composition according to Claim 1 comprising 6-p-chloroanilino-4-methyl-1,3,2-benzothiazathiolium chloride.

3. A method of producing an antiallergic effect in a mammal which comprises administering to said mammal a therapeutically effective dose of a compound of Formula I.

4. A method of producing an antiallergic effect in a mammal which comprises administering to said mammal a therapeutically effective dose of 6-p-chloro-anilino-4-methyl-1,3,2-benzothiazathiolium chloride.

5. 6-p-Chloroanilino-4-methyl-1,3,2-benzothiazathiolium chloride.

6. 6-p-Hydroxyanilino-4-methyl-1,3,2-benzothia-zathiolium chloride.

7. A pharmaceutical composition comprising a compound of the Formula

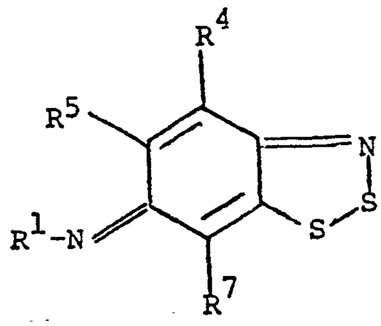

wherein

$R^1$ is an optionally substituted hydrocarbyl group and

$R^4$, $R^5$ and $R^7$ are independently hydrogen atoms or substituents

or one of its physiologically acceptable acid addition salts.

8. A pharmaceutical composition according to Claim 7 comprising 6-p-chlorophenylimino-4-methyl-6H-1,2,3-benzodithiazole or one of its physiologically acceptable acid addition salts.

9. 6-p-Chlorophenylimino-4-methyl-6H-1,2,3-benzodithiazole and its physiologically acceptable acid addition salts.